Europäisches Patentamt

European Patent Office

Office européen des brevets

(11) Numéro de publication: **0 109 873**
**B1**

(12) **FASCICULE DE BREVET EUROPEEN**

(45) Date de publication du fascicule du brevet:
**06.08.86**

(51) Int. Cl.⁴: **A 61 K 31/70** // (A61K31/70,
31:415, 31:07)

(21) Numéro de dépôt: **83402044.8**

(22) Date de dépôt: **21.10.83**

(54) Composition thérapeutique à base d'ADN et d'allantoïne utile notamment pour la cicatrisation des plaies.

(30) Priorité: **18.11.82 FR 8219294**

(43) Date de publication de la demande:
**30.05.84 Bulletin 84/22**

(45) Mention de la délivrance du brevet:
**06.08.86 Bulletin 86/32**

(84) Etats contractants désignés:
**BE CH DE GB IT LI LU NL**

(56) Documents cités:
**US - A - 3 326 892**

**CHEMICAL ABSTRACTS, vol. 63, no. 4, 16 août 1965,
colonne 4096h - 4097a, Columbus, Ohio, US, I.I.
LUBOWE et al.: "Allantoin complexes for topical use"**

(73) Titulaire: **Ranson, Michèle, 7, place du Général Catroux,
F-75017 Paris (FR)**

(72) Inventeur: **Ranson, Michèle, 7, place du Général
Catroux, F-75017 Paris (FR)**

(74) Mandataire: **Ores, Irène et al, CABINET ORES 6, Avenue
de Messine, F-75008 Paris (FR)**

ACTORUM AG

## Description

La présente invention est relative à une nouvelle composition thérapeutique particulièrement efficace dans la cicatrisation des plaies.

La présente invention s'est fixée pour but de pourvoir à une composition thérapeutique qui assure la réparation des pertes de substance cutanée, lorsque le processus de cicatrisation physiologique est perturbé, c'est-à-dire retardé ou inexistant.

Après de longues recherches (notamment cliniques), la Demanderesse a pu mettre au point une composition à activité cicatrisante adaptée aux traitements des plaies à évolution lente et à cicatrisation difficile.

La présente invention a pour objet une nouvelle composition thérapeutique utile pour le traitement des plaies, caractérisée en ce qu'elle est constituée d'une association d'allantoïne avec l'acide désoxyribonucléique (ADN). Il était déja connu, notamment par le brevet US-A-3 326 892 d'utiliser le produit de la réaction de l'allantoïne sur le RNA (sel de sodium) à des fins cosmétiques.

Suivant un mode de réalisation avantageux de l'objet de l'invention, le rapport pondéral allantoïne/ADN est compris entre 1/2 et 1/8.

Suivant un autre mode de réalisation avantageux de l'objet de l'invention, la composition comprend en outre 5 000 à 20 000 U.I. de vitamine A pour 1 g de ladite composition.

Selon un autre mode de réalisation particulièrement avantageux, l'ADN utilisé est un ADN hautement polymérisé.

Conformément à l'invention, la nouvelle composition, qui peut se présenter sous forme de poudre, gel, compresse stérile prête à l'emploi, collyre, ou solution nasale, contient entre 2 à 100% (poids/poids) en principes actifs.

Outre les dispositions qui précèdent, l'invention comprend encore d'autres dispositions qui ressortiront de la description qui va suivre, qui se réfère à un exemple de préparation, ainsi qu'à un compte-rendu clinique.

Il doit être bien entendu, toutefois, que les exemples et compte rendu sont données uniquement à titre d'illustration de l'objet de l'invention dont ils ne constituent, en aucune manière, une limitation.

### Exemple de préparation

On introduit dans une cuve en acier inoxydable de 100 ℓ thermostatée, 35 ℓ d'eau distillée chauffée à 65°C. On ajoute ensuite sous agitation par petites fractions 0,2 kg d'allantoïne. Après dissolution totale on verse en pluie 1 kg d'ADN hautement polymérisé préalablement broyé (taille moyenne 250 µm). On agite énergiquement, puis la dissolution effectuée, on complète le volume à 40 litres par de l'eau distillée chauffée à 65°C, puis on maintient encore l'agitation pendant 30 minutes.

La solution ainsi obtenue est filtrée stérilement et répartie en conditionnement unitaire.

### Exemples de conditionnements utilisés

- Compresses de gaze tissées ou tressées stériles comportant 5 ml de gel pour une surface de 2,5 cm² et conditionées en sachets aluminés stériles.
- Flacons stilligouttes de 50 ml pour collyres.
- Tubes individuelles stériles de volume utile de 10 ml.
- Flacons pulvérisateurs stériles de 10 ml munis d'une pompe mécanique (ou tout conditionnement approprié pour usage O.R.L.).

### Compte-rendu clinique

L'expérimentation clinique a porté sur l'activité cicatrisante comparée de trois groupes de compresses impregnées respectivement:

- d'un gel d'ADN hautement polymérisé à 2,5% (produit A);
- d'une solution d'allantoïne à 0,5% (produit B);
- d'un gel conforme à la présente invention (2,5% d'ADN et 0,5 d'allantoïne: produit C).

L'expérimentation a été conduite sur 30 malades répartis en trois groupes de 10.

La population traitée était homogène: il s'agissait de malades présentant des ulcères d'origine veineuse: lésions de taille et de situation identiques (diamètre compris entre 1,5 et 3 cm, malléolaires et plantaires) la plupart des lésions évoluant depuis plusieurs semaines: 6 mois pour 5 cas, plus d'un an pour 2 cas. Les malades ont été répartis en fonction de la taille et de l'ancienneté des lésions pour avoir une norme égale dans les 3 groupes traités.

La thérapeutique générale a été identique et limitée à la contention.

Les lésions traitées ne présentaient pas de caractères inflammatoires importants, peu ou pas douloureuses, sans aucun signe d'infection à distance, les malades étant apyrétiques.

Le traitement a consisté à appliquer deux fois par jour des compresses largement imprégnées après désinfection par une solution antiseptique. Les compresses laissées au contact de la plaie ont été entourées d'une bande fine.

Les résultats sont consignés dans le tableau I suivant:

### TABLEAU 1

| Résultats | Compres-ses A | Compres-ses B | Compres-ses C |
|---|---|---|---|
| T.B. | 5 | 1 | 7 |
| B. | 2 | 1 | 7 |
| N.S. | 3 | 8 | 1 |
| Total des malades | 10 | 10 | 10 |

Nombre total de malades traités: 30
Nombre de cas pour chaque type de compresses: 10
Appréciation des résultats:
T.B. = cicatrisation totale;
B. = diminution du diamètre de la plaie, apparition d'un bourrelet épidermique périphérique;
N.S. = pas d'amélioration sensible.

En conclusion:

Sur les trois groupes de 10 malades traités, on a obtenu:

- avec les compresses A: 5 + 2, soit 7 résultats positifs;
- avec les compresses B: 2 résultats positifs seulement;
- avec les compresses C: 9 résultats positifs.

On voit donc que l'action positive déjà obtenue avec l'ADN seul (7/10) est améliorée si on associe l'allantoïne (9/10). Il est à noter que les applications ont été pratiquées dans tous les cas trente jours de suite et que la cicatrisation a été obtenue en moyenne en vingt jours avec la composition conforme à la présente invention. En ce qui concerne le résultat non significatif (N.S.), c'est-à-dire n'ayant pas cicatrisé en trente jours, on a prolongé le traitement deux semaines supplémentaires et on a obtenu une ébauche de cicatrisation après 30 + 15, soit quarante-cinq jours.

Le total de 9 résultats positifs sur 10, le dernier étant en voie de cicatrisation, montre clairement la supériorité de la composition conforme à la présente invention sur chacun des composants employés seuls.

Au cours de divers traitements cliniques, la Demanderesse a également constaté une action nette de la nouvelle composition sur les escarres, sur les crevasses du sein, sur les plaies traumatiques, sur les érythèmes fessiers et sur les brûlures.

Ainsi que cela ressort de ce qui précède, l'invention ne se limite nullement à ceux de ses modes de réalisation et d'application qui viennent d'être décrits de façon plus explicite.

**Revendications**

1. Composition thérapeutique utile pour le traitement des plaies, caractérisée en ce qu'elle est constituée d'une association d'allantoïne avec l'acide désoxyribonucléique (ADN).

2. Composition selon la revendication, caractérisée en ce que le rapport pondéral allantoïne/ADN est compris entre 1/2 à 1/8.

3. Composition selon l'une quelconque des revendications 1 et 2, caractérisée en ce qu'elle comprend en outre 5000 à 20000 U.I. de vitamine A pour 1 g de ladite composition.

4. Composition selon l'une quelconque des revendications 1 à 3, caractérisée en ce que l'ADN utilisé est un ADN hautement polymérisé.

5. Composition selon l'une quelconque des revendications 1 à 4, caractérisée en ce qu'elle contient 2 à 100% (poids/poids) en principes actifs.

**Patentansprüche**

1. Therapeutische Zusammensetzung für die Behandlung von Wunden, dadurch gekennzeichnet, dass sie aus einer Assoziation von Allantoin mit Desoxyribonucleinsäure /DNA) besteht.

2. Zusammensetzung nach Anspruch 1, dadurch gekennzeichnet, dass das Gewichtsverhältnis Allantoin/DNA 1/2 bis 1/8 beträgt.

3. Zusammensetzung nach einem der Ansprüche 1 und 2, dadurch gekennzeichnet, dass sie weiterhin 5000 bos 20000 I.E. Vitamin A pro 1 g der genannten Zusammensetzung enthält.

4. Zusammensetzung nach einem der Ansprüche 1 bis 3, dadurch gekennzeichnet, dass die verwendete DNA eine hochpolymerisierte DNA ist.

5. Zusammensetzung nach einem der Ansprüche 1 bis 4, dadurch gekennzeichnet, dass sie 2 bis 100% (Gewicht/Gewicht) Wirkstoffe enthält.

**Claims**

1. Therapeutic composition useful for the treatment of wounds, characterized in that it is constituted by an association of allantoin with desoxyribonucleic acid (DNA).

2. Composition according to claim 1, characterized in that the ratio by weight allantoin/DNA is comprised between 1/2 and 1/8.

3. Composition according to any one of claims 1 and 2, characterized in that it comprises in addition 5000 to 20000 I.U. of vitamin A per 1 g of said composition.

4. Composition according to any one of claims 1 to 3, characterized in that the DNA used is a highly polymerised DNA.

5. Composition according to any one of claims 1 to 4, characterized in that it contains 2 to 100% (weight/weight) of active principles.